# EUROPEAN PATENT SPECIFICATION

(11) **EP 3 553 045 B1**
(45) Date of publication and mention of the grant of the patent: **04.09.2024**
(21) Application number: 18166315.4
(22) Date of filing: 09.04.2018
(51) Int. Cl.: C07C 51/38, C07C 57/04, B01J 23/58, B01J 23/44, B01J 35/30, B01J 35/61, B01J 35/63, C01F 7/168

(54) **PROCESS TO PRODUCE METHACRYLIC ACID MONOMER FROM BIOMASS-DERIVED CARBOXYLIC ACIDS**
VERFAHREN ZUR HERSTELLUNG VON METHACRYLSÄUREMONOMER AUS VON BIOMASSE STAMMENDEN CARBONSÄUREN
PROCÉDÉ POUR PRODUIRE UN MONOMÈRE D'ACIDE MÉTHACRYLIQUE À PARTIR D'ACIDES CARBOXYLIQUES ISSUS DE LA BIOMASSE

(43) Date of publication of application: 16.10.2019
(73) Proprietor: Kemijski Institut, 1000 Ljubljana (SI); Helios TBLUS d.o.o., 1230 Domzale (SI)
(72) Inventor: Likozar, Blaz, 1000 Ljubljana (SI); Bohre, Ashish, 1000 Ljubljana (SI); Grilc, Miha, 1000 Ljubljana (SI); Ocepek, Martin, 1230 Domzale (SI); Venturini, Peter, 1230 Domzale (SI); Steinbücher, Miha, 1230 Domzale (SI)
(74) Representative: Grünecker Patent- und Rechtsanwälte PartG mbB

(56) References cited:
- WO-A1-2015/030580
- US-A1- 2005 232 857
- US-A1- 2013 116 117
- MARYAM PIRMORADI ET AL: "Synthesis of Methacrylic Acid by Catalytic Decarboxylation and Dehydration of Carboxylic Acids Using a Solid Base and Subcritical Water", ACS SUSTAINABLE CHEMISTRY & ENGINEERING, vol. 5, no. 2, 30 December 2016 (2016-12-30), US, pages 1517 - 1527, XP055513425, ISSN: 2168-0485, DOI: 10.1021/acssuschemeng.6b02201
- JÉRÔME LE?NÔTRE ET AL: "Synthesis of Bio-Based Methacrylic Acid by Decarboxylation of Itaconic Acid and Citric Acid Catalyzed by Solid Transition-Metal Catalysts", CHEMSUSCHEM, vol. 7, no. 9, 13 July 2014 (2014-07-13), DE, pages 2712 - 2720, XP055513370, ISSN: 1864-5631, DOI: 10.1002/cssc.201402117

## Description

### SUMMARY OF THE INVENTION

The present invention relates to the developing of a sustainable and selective catalytic process for methacrylic acid synthesis from inexpensive and abundant biomass derived feedstocks to reduce the overall production cost and provide benefits to pertinent industries and society. Accordingly the present invention provides a sustainable catalytic process for production of methacrylic acid from biomass derived feedstocks. The method comprises the decarboxylation of bio-based carboxylic acids at temperature range from 150-300 °C and pressure range from 5 to 100 bar over heterogeneous catalysts.

### BACKGROUND OF THE INVENTION

Methacrylic acid (MAA) is an industrially important monomer, mostly used for the production of plastics, optical glasses, lenses, moldings, fibers, resins etc. Copolymers of MAA are also essential components found in surface coatings, paints, adhesives, and emulsion polymers. The properties of MAA derived polymers include good mechanical strength, scratch resistant and outstanding optical properties.

Currently, the majority of MAA in the industry is produced from petrochemical resources through the acetone-cyanohydrin (ACH) process. This method relies on expensive and extremely toxic feedstocks and corrosive concentrated acids. Besides the use of harmful substrates, low atom economy, poor product selectivity and the net emission of greenhouse gases are other drawbacks, associated with this industrial process, while the production is based exclusively on a non-renewable fossil-based resource. An improved process (Alpha process) to produce MAA involves catalytic carbonylation of petroleum-derived ethylene to methyl propionate followed by condensation of methyl propionate with formaldehyde to produce MAA. However, the Alpha process requires the provision of multiple feedstocks in one location, which can lead to availability issues. Considerations of the disadvantages associated with current industrial-scale processes have driven the ongoing research and development toward developing sustainable processes for MAA production from renewable carbon sources. Currently however no commercial routes to produce MAA from bio-based resources are available, but several attempts have been made and potential pathways for bio-based MAA have been reported.

Fermentation of renewable carbohydrates by using micro-organisms is an interesting greener production route for MAA. In this regards, WO2012135789A discloses a biotechnological process for the synthesis of MAA from biomass derived glucose using a recombinant microbe introducing with non-natural occurring plural enzyme genes. Smith et al., (BioorganicMed. Chem. 1994, 2(7), 589-594*)* has utilized a naturally occurring photosynthetic microbe to produce MAA from 3-mercaptoisobutyric acid. Further, in US2009/013079A1 a method of producing MAA employing the activity of a biocatalyst in the presence of methacrylyl-CoA and an alcohol is disclosed. Interestingly, Pyo et al., (Green Chem., 2012, 14, 1942-1948*)* proposed a two-step process for the production of MAA by coupling biotransformation and catalytic routes. 2-methyl-1,3-propanediol (2M1,3PD) was first oxidized to 3-hydroxy-2-methylpropionic acid over *Gluconobacter oxydans,* and then subsequently dehydrated to methacrylic acid using a TiO₂ catalyst. Generally, however, industrial processes, in particular for standard and mass educts, such as MAA require high product selectivity and low production cost. The biotransformation routes are not cost effective and the separation of side product makes this process even more expensive. Additionally, it was found that MAA is toxic to genetically engineered microorganisms therefore these pathways are not currently technically viable.

Catalysis is regarded as a key enabling technology for biomass conversion in general, and for improving the yield and selectivity of MAA production. Carlsson et al., (Ind. Eng. Chem. Res. 1994, 33, 1989-1996) has disclosed a method to produce MAA from the homogenous mineral base catalyzed decarboxylation of citric acid at high temperatures of 360 °C and with a maximum yield of 70%. Citric acid is present naturally in the juices of citrus fruits, as well as in agro-industrial by-products or residual products, such as sugarcane molasses or vinasse. EP2643283B1 discloses the preparation of MAA and its derivatives from biomass derived dicarboxylic acids in the presence of base catalysts. US patent 9096512B2, uses a 3 step process to prepare MAA from biomass derived isobutyraldehyde and isobutanol. In the first step isobutyraldehyde or isobutanol is oxidized to isobutyric acid. Oxidizing agents are selected from ozone, nitric acid, potassium permanganate, chromic acid and chromium trioxide. In the second step isobutyric acid is halogenated to 3-haloisobutyric acid. The halogen is selected from chlorine, fluorine, bromine and iodine. Finally, 3-haloisobutyric acid is dehalogenated to MAA in the presence of sodium hydroxide or potassium hydroxide. Most recently US patent 9751823B2, described a process for making MAA via methacrolein from bio-based isobutene, wherein the bio-based methacrolein is prepared from ethanol or from acetic acid in the presence of a ZnₓZr_{y}O_{z} mixed oxide catalyst. J. Le Nôtre et al. (ChemSusChem 2014, 7 (9), 2712-2720) has reported that MAA could be synthesized by the decarboxylation of itaconic acid (IA) using solid-transition metal catalysts. The best result was obtained at 250 °C with 84 % of MAA yield over a Pt/Al₂O₃ catalyst. However, a liquid base additive is required with Pt based catalysts and this is a factor, which makes this process non-greener.

M. Pirmoradi et al., ACS Sustainable Chem. Eng. 2017, 5, 1517-1527 reports on the synthesis of methacrylic acid from the biobased substrates citric acid, itaconic acid, and 2-hydroxyisobutyricacid (2-HIBA). Hydrotalcite, a solid base catalyst, was employed to form methacrylic acid (MAA) through decarboxylation of itaconic acid and citric acid. Optimum MAA yields occurred at a substrate to catalyst mass ratio of 9.6 g-substrate/g-catalyst and 21% for citric acid and 6.4 g/g and 23% for itaconic acid (250°C, 15 min). Catalyst reusability experiments resulted in higher methacrylic acid yields for both citric and itaconic acid. Methacrylic acid was also formed from 2-hydroxyisobutyric acid in a single-step dehydration reaction. Among these three substrates, the highest yield of methacrylic acid (71.5%) was achieved at 275°C (1 min) using 2-HIBA and subcritical water. Finally, the conversion of these three acids in a simulated residual fermentation broth (0.1 M NaOH, 0.04 M Na2SO4, 0.04 M Na2HPO4, 0.06 M glucose,0.12 wt % albumin) was tested and MAA yields from itaconic acid and citric acid using hydrotalcite increased in the presence of these fermentation"impurities"and decreased slightly from 2-HIBA.

### OBJECT OF THE PRESENT INVENTION

Currently, the hazardous acetone-cyanohydrin process is the main route for producing MAA. Toxic starting materials, high process cost, and the large amount of bisulfate waste are problems with this process. Besides the use of harmful substrates, low atom economy, poor product selectivity and the net emission of greenhouse gases are other drawbacks, associated with the industrial process. Natural resources are being consumed in the industrial production of MAA; from an environmental and economic standpoint, it is important to recoup them in as great extent as possible. The use of petroleum based raw products is a major drawback, which has to be overcome. It would be desirable to have a simple and practicable synthetic route to produce MAA on an industrial scale starting from non-petroleum sources. The development of such processes would be a maj or breakthrough, complementing the shift of the world economy from the eventually depleting petrochemical feedstock to biomass-based resources. Such an advance might impact a relief from the volatility of the global MAA prices and drive toward a more sustainable technological solution.

In this context it would be advantageous to be able to provide a process as outlined above based on the use of sustainable feedstocks such as citric acid, itaconic acid, aconotic acid, mesaconic acid, and citraconic acid for the production of MAA. Citric acid is a widely available renewable resource produced via high yield fermentation process. Aconitic acid is the dehydration product of citric acid. Itaconic acid is produced on an industrial scale by the fermentation of biomass with Aspergillus terreus fungus frequently found in the nature. Itaconic acid can also be produced from the dehydration and decarboxylation of citric acid. Itaconic acid play a central role in the bio-based chemicals portfolio, as evidenced by their prevalence in the top 12 chemicals from biomass identified by the U.S. Department of Energy. Mesaconic acid and citraconic acid are the isomers of itaconic acid.

The industrial route for MAA production requires harsh reaction conditions (high temperature and pressure) that produce a significant amount of by-products which may be difficult to remove and cause further purification and processing problems. In this context, it would be advantageous if one could provide an efficient synthetic process that can replace the current multiple-step and energy-intensive industrial process of the MAA production with relatively mild operating conditions in order to achieve both a high yield and selectivity.

In the context of the prior art methods disclosed above it would also be advantageous if one could provide a process not requiring the use of homogeneous catalysts or catalysts requiring the presence of problematic co-catalysts, such as liquid bases, as these prior art catalysts give rise to problems in relation with catalyst removal, side- and by-product removal and furthermore often cannot be reused at all.

### SUMMARY OF THE PRESENT INVENTION

The present invention solves the problems outlined above and provides the method as defined in claim 1, as well as the use of a catalyst in the method of claim 1 defined in claim 7. Preferred embodiments of the method and the use of a catalyst are disclosed in claims 2 to 6 and 8 to 9 respectively, as well as in the following description.

### BRIEF DESCRIPTION OF THE FIGURES

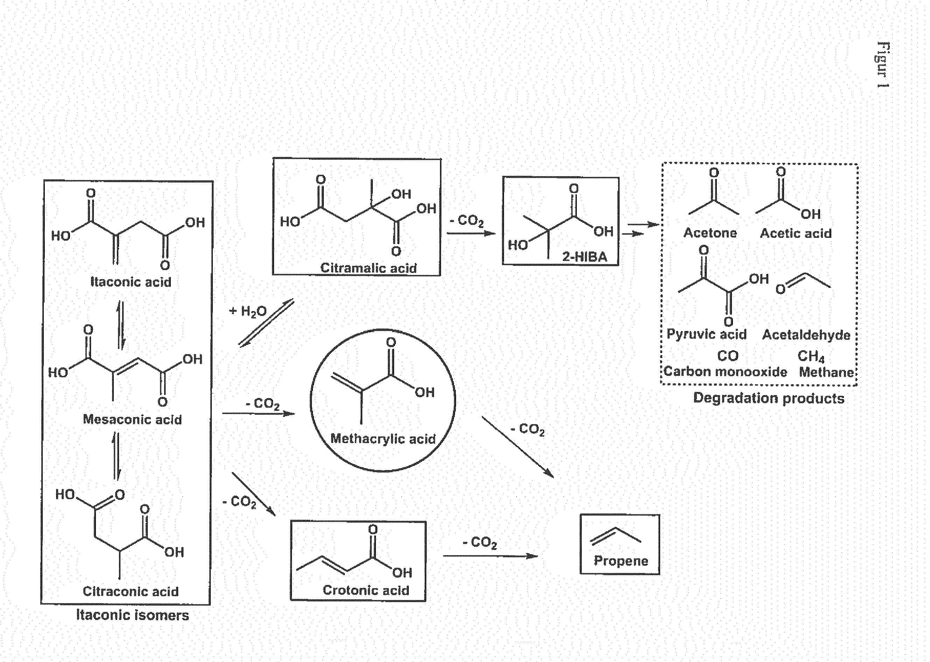

FIG. 1 depicts the possible reaction pathways that occur during the decarboxylation of itaconic acid.
FIG. 2 shows the comparison of the activity of the Pd@BHA catalyst with other catalysts in the decarboxylation of IA to MAA, under the following reaction conditions: Itaconic acid (2 g), water (150 mL), catalyst (1 g), initial pressure (20 bar), t (3 h), T (250 °C).
FIG. 3 shows the comparison of the activity of the BHA catalyst with other catalysts for the decarboxylation of IA to MAA, under the following reaction conditions: Itaconic acid (2 g), water (150 mL), catalyst (1 g), initial pressure (20 bar), t (3 h), T (250 °C).
FIG. 4a shows the time dependent formation of MAA from IA decarboxylation with the Pd@BHA catalyst, under the following reaction conditions: Itaconic acid (2 g), water (150 mL), catalyst (1 g), initial pressure (20 bar).
FIG. 4b shows the time dependent formation of MAA from IA decarboxylation with the BHA catalyst, under the following reaction conditions: Itaconic acid (2 g), water (150 mL), catalyst (1 g), initial pressure (20 bar).
FIG.5 is a wide range angle XRD pattern of the Pd@BHA catalyst.
FIG.6 shows the temperature programmed desorption of carbon dioxide of the Pd@BHA catalyst.
FIG 7a shows the SEM image of the Pd@BHA catalyst.
FIG. 7b shows the EDS spectrum of the Pd@BHA catalyst.
FIG.8 shows the shows the results of the recyclability study of the Pd@BHA for the decarboxylation of IA to MAA, under the following reaction conditions: Itaconic acid (2 g), water (150 mL), catalyst (1 g), initial pressure (20 bar), t (3 h), T (250 °C).
FIG.9 shows the shows the results of the recyclability study of the BHA for the decarboxylation of IA to MAA, under the following reaction conditions: Itaconic acid (2 g), water (150 mL), catalyst (1 g), initial pressure (20 bar), t (3 h), T (250 °C).
FIG. 10 shows the HPLC chromatogram of sample with injection volume 1 (µL) (a) and 50(µL) (b) after decarboxylation of IA over BHA catalyst.

### DETAILED DESCRIPTION OF THE INVENTION

The present invention is based on a judiciously designed heterogeneous catalyst for the decarboxylation of bio-based feedstocks. In most of the reported routes to MAA homogeneous base catalysts have been utilized but these catalysts have disadvantages in relation to separation, recyclability and disposal. Heterogeneous catalytic systems, on the other hand, offer distinct advantages in terms of cost reduction, high activity and selectivity easy separation and reusability. Another merit of heterogeneous catalysts is their stability to high temperature and pressure.

The present invention accordingly provides a process for the decarboxylation of bio-based feedstocks to MAA with a heterogeneous catalytic system that offers high selectivity and yield under relatively mild reaction conditions. As defined in claims 1 and 7, the heterogeneous catalyst to be employed in the present invention is a catalyst not requiring any base co-catalyst, in liquid or other form, as required in the prior art using a Pt catalyst supported on Al₂O₃. The heterogeneous catalyst of the present invention is a catalyst which in particular enables production of MAA under relatively mild conditions and furthermore allows an easy separation of the used catalyst from the reaction mixture. In particular it has been shown that the catalysts of the present invention can be reused, in many instances even without any re-activation (for example by reductive treatments to remove any undesired oxidation of the transition metals, if contained in the catalysts or other conventional processes known to the skilled person to re-activate a spent catalyst). The catalyst in accordance with the present invention is not a hydrotalcite. Preferred examples of catalysts are listed in the following:
lanthanum hexa-aluminate, barium hexa-aluminate (BHA), sodium hexa-aluminate, calcium hexa-aluminate, magnesium hexa-aluminate;
transition metal supported catalysts, preferably transition metals selected from ruthenium (Ru) or palladium (Pd), wherein the support is stable to the reaction conditions, with the support being barium hexa-aluminate, with preferred amounts of transition metal ranging from 1 to 10 weight percent relative to the support.

The catalysts are based on hexa-aluminate. Accordingly, the catalysts to be employed in the present invention comprise a hexa-aluminate.

It is to be understood that preferred embodiments described for the catalyst as such of course also are applicable to the use of the catalyst in the process of the present invention.

Examples of suitable hexa-aluminate based catalysts are lanthanum hexa-aluminate, barium hexa-aluminate (BHA), sodium hexa-aluminate, calcium hexa-aluminate, magnesium hexa-aluminate, preferably BHA, as well as transition metal based catalysts, wherein the transition metal is supported on a hexa-aluminate, preferably selected among those exemplified above. The transition metals to be employed in accordance with the present invention are preferably selected among Ru, Rh, Pd, Pt, most preferably Pd.

The catalysts in accordance with the present invention preferably are barium hexa-aluminate and transition metals supported on this material. The suitable amount of transition metal in the catalysts of the present invention is in the range of from 1 to 10 weight percent relative to the support. The BET specific surface area of the catalysts of the present invention is generally from 0.01 to 200 m² /g, preferably from 0.1 to 150 m² /g, in particular from 1 to 100 m² /g. The pore volume preferably is between 0.1 to 0.50 cm³ per gram, preferably between 0.15 to 0.40 cm³ per gram. In a preferred embodiment the BET surface is from 0.1 to 150 m², preferably from 1 to 100 m², in combination with a pore volume of between 0.1 to 0.50 cm³ per gram, preferably between 0.15 to 0.40 cm³ per gram. BET specific surface area and pore volume are determined according to the methods disclosed in Lowell, S., Shields, J.E., Thomas, M.A., and Thommes, M., (2012). Characterization of porous solids and powders: surface area, pore size and density (Vol. 16), Springer Science & Business Media).

The catalysts disclosed herein, in particular the hexa-aluminate catalytic system disclosed herein has particularly shown to efficiently decarboxylate biomass derived feedstocks. Without being bound to the following explanation, it may be that the superior and unexpected activity is based on the fact that the surface of hexa-aluminate catalysts have a strong basic character, probably due to the preferential exposition at the crystallite surface of the so-called "mirror planes" containing the barium ions. The M²⁺-O²⁻ couples in hexa-aluminate catalyst are directly bonded to the alumina blocks at the crystallite surface. The "layered structure of aluminate" provides stability to hexa-aluminate catalyst and the Lewis acid sites (Al³⁺) may also contribute to the decarboxylation step. The excellent activity of the hexa-aluminate catalyst accordingly may be attributed to the high basicity and layered structure that facilitates the dissociation of carboxylic acid to the corresponding carboxylate anions. In any case, the hexa-aluminate catalytic system disclosed here shows very high activity, selectivity and yield of MAA from bio-based feedstocks - higher than other catalysts tested or reported in the literature for the decarboxylation of bio-based feedstocks to MAA.

In order to demonstrate the superiority of the present invention, these following catalysts have been evaluated in relation with the catalytic conversion to MAA, including dolomite, sodium silicate, gamma alumina, zeolite-Y, barium oxide, hydromagnesite, perovskite (calcium titanate), NaY zeolite, NaX zeolite, zeolites CBV-300, zeolites CBV-500, zeolites CBV-712, zeolites CBV-720, zeolites CBV-760, zeolites CBV-780, zeolites CBV-3024E, zeolites CBV-8014, Mg-Al hydrotalcite (Mg/Al molar ratios 0.5, 1 and 2), Ca-Mg-Al hydrotalcite, Mg-Zr mixed oxide (Mg/Zr molar ratios 0.5, 1 and 2), Mg-Fe mixed oxide (Mg/Fe molar ratios 0.5, 1 and 2), La-Mg mixed oxide (La/Mg molar ratios 0.5, 1 and 2), Ca-Mg mixed oxide (Ca/Zr molar ratios 0.5, 1 and 2), Ca-Zr mixed oxide (Ca/Zr molar ratios 0.5, 1 and 2), Ca-Zn mixed oxide (Ca/Zn molar ratios 0.5, 1 and 2), TiO₂-MgO mixed oxide (Ti/Mg molar ratios 0.5, 1 and 2), zirconium phosphate and MgO-SnO₂ catalysts, transition metal supported catalysts with transition metals selected from ruthenium (Ru) or palladium (Pd) provided on high surface area catalyst supports, selected among carbon, and aluminum oxide. The respective results, some of which are discussed further in relation with experimental data shown below, prove that the catalysts in accordance with the present invention enable the preparation of the desired target molecule in a highly effective manner, thereby overcoming the drawbacks associated with the prior art and solving the problems outlined above.

Accordingly the process in accordance with the present invention comprises the step of converting a starting material, selected among citric acid, aconitic acid, itaconic acid, mesaconic acid, and citraconic acid, preferably obtained from renewable resources (bio-based) to MAA in the presence of a catalyst as defined above, selected among catalysts based on hexa-aluminate, preferably selected among lanthanum hexa-aluminate, barium hexa-aluminate (BHA), sodium hexa-aluminate, calcium hexa-aluminate, magnesium hexa-aluminate, preferably BHA, as well as transition metal based catalysts, wherein the transition metal is supported on a hexa-aluminate, preferably selected among those exemplified above. As for the catalyst as such the transition metals to be employed in accordance with the present invention are preferably selected among Ru, Rh, Pd, Pt, most preferably Pd.

The starting materials to be converted in accordance with the present invention typically are provided in a polar solvent, preferably water. It has been found that the concentration of the starting material in the polar solvent preferably is at least 0.01 M and not more than 1 M, such as from 0.05M to 0.5M, preferably 0.1M to 0.3 M, such as 0.1m, 0.2M, 0.3M, or 0.4M. It has been found that the amount of catalyst in the reaction mixture (calculated for a batch reaction, suitable adaptations have to be made for continuous processes) is not less than 10 mass percent and not higher than 150 mass percent with respect to substrate (i.e. the above identified starting material), such as from 25 to 100 mass percent.

Preferably the reaction is carried out under inert gas, and nitrogen gas is a preferred example of a suitable inert gas, due to its abundance and low cost.

The process disclosed here typically is carried out at a temperature of from 150°C to 300°C, preferably from 200°C to 275°C, more preferably from 225°C to 260°C, such as 250°C. The pressure typically is in the range of from 5 to 100 bar, preferably from 10 to 70 bar and more preferably from 10 to 40 bar, and in particular 30 bar or less and 15 bar or more, such as 20 bar.

A preferred combination of reaction conditions is a temperature of from 225°C to 260°c and a pressure of from 15 to 30 bar.

The reaction time is not critical and may depend from the desired balance of conversion and efficiency as well as selectivity, but generally reaction times of form 30 to 1000 min are suitable, such as from 60 to 600 min, from 60 to 300 min and in embodiments from 80 to 200 min.

The reaction may be carried out in any suitable reactors known to the skilled person, but stainless steel reactors (autoclave) are in particular suitable. However, other materials may also be suitable as reactor materials and it is of course also possible to provide the inner surface of such a reactor, completely or partially with a coating of a material inert towards the reaction, such as a polymer coating, a ceramic coating etc.

It is to be understood, that the various process parameters, such as starting material concentration, type of polar solvent, catalyst amount, temperature, pressure and reaction time (residence time), while being disclosed individually, are considered in the context of the present invention in combination, such as a process employing water as solvent, a starting material concentration of from 0.05M to 0.5M, a catalyst amount of from 25 to 100 mass percent, a reaction temperature of from 200°C to 275°C, preferably from 225°C to 260°C, a pressure of from 10 to 40 bar, preferably from 15 to 30 bar and a reaction time of from 60 to 300 min. However, all other combinations possible from the ranges and preferred embodiments are also comprised by and disclosed in this invention.

In the examples described herein the catalytic decarboxylation of all substrates was performed using a stainless steel high pressure reactor under the standard operation conditions. The novel process of the present invention was carried out at a temperature greater than 150 °C, most preferably between 150 °C and 300 °C, especially between 200°C and 275°C. The reactions were carried out under pressure. Preferably, the processes are carried out at pressures of at least 10 bar of N₂, more preferably at least 20 bar, in some embodiments at least 30 bar, in some embodiments pressure is in the range of 10 bar to 70 bar. In general, the reaction should be conducted under conditions where the residence time of the feedstock solution over the catalyst is appropriate to generate the desired products. Preferably, the reaction time is in the range of between 60 and 500 minutes, typically 60-300 minutes, more preferably 60-250 minutes, most preferably 80-200 minutes.

The following table (Table 1) summarizes experimental results obtained in accordance with the present invention.

| **Table 1**. Screening of reaction conditions for decarboxylation of itaconic acid with Pd@BHA catalyst. | | | | | | | | | |
|---|---|---|---|---|---|---|---|---|---|
| **Entry^{[a]}** | **T[°C]** | **P[bar]** | **IA [M]** | **Conversion [%]** | **Selectivity [mol %]** | | | | |
| | | | | | **MAA** | **Isomers^{[b]}** | **2-HIBA** | **AA** | **Others^{[c]}** |
| 1 | 175 | 40 | 0.15 | 78 | 5 | 24 | - | 15 | 56 |
| 2 | 200 | 40 | 0.15 | 84 | 8 | 21 | 2 | 20 | 49 |
| 3 | 225 | 40 | 0.15 | 90 | 26 | 14 | 10 | 18 | 32 |
| 4 | 250 | 40 | 0.15 | 100 | 30 | 2 | 9 | 14 | 45 |
| 5 | 275 | 40 | 0.15 | 100 | 24 | - | 7 | 10 | 59 |
| 6 | 250 | 10 | 0.15 | 100 | 22 | 2 | 9 | 24 | 43 |
| 7 | 250 | 20 | 0.15 | 100 | 37 | 2 | 11 | 21 | 29 |
| 8 | 250 | 20 | 0.19 | 95 | 30 | 5 | 11 | 23 | 31 |
| 9 | 250 | 20 | 0.10 | 100 | 46 | 3 | 12 | 26 | 13 |
| 10^{[d]} | 250 | 20 | 0.10 | 87 | 28 | 12 | 11 | 15 | 34 |
| [a] Reaction conditions : Pressure reactors, IA, solvent (water), time (3 h), Pd@BHA catalyst (1 g, 10 wt % of Pd), [b] Isomers = mesaconic acid + citraconic acid, [c] Others = pyruvic acid, crotonic acid, acetone, propene, carbon dioxide and carbon monoxide. [d] No catalyst. Values were determined by HPLC. IA is itaconic acid, MAA is methacrylic acid, AA is acetic acid and 2-HIBA is 2-hydroxyisobutyric. | | | | | | | | | |

Results are shown in table 1 for the palladium supported barium hexa-aluminate (Pd@BHA) catalyzed decarboxylation. The effect of the reaction temperature on the performance of the Pd@BHA catalyst was investigated in the temperature range of 175-275 °C (Table 1, entry 1-5). At low temperatures (175-200 °C) the conversion of IA is incomplete and selectivity of MAA is low, however still better compared with many prior art approaches. It appears that degradation had already occurred from the observation of the high amount of byproducts at low temperatures. Upon increasing the reaction temperature from 200 to 250 °C the selectivity of MAA was increased from 8 to 30 % with complete conversion of IA. Above 250 °C parasitic reactions begin to predominate due to higher acidity of the subcritical reaction medium that reduces the MAA selectivity. These results prove the possibility to carry out the process of the present invention over a broad temperature range, with options to maximize conversion and selectivity by adjusting the temperature to the more preferred ranges described herein.

When the reaction was performed at low pressure (10 bar) the selectivity of MAA was decreased (Table 1, entry 6). When the pressure was increased from 10 bar to 20 bar under these conditions, the decarboxylation rate increased. This resulted in an even more enhanced yield of MAA of up to 37% (Table 1, entry 7). High concentration of IA (0.19) results in a lower yield of MAA. (Table 1 entry 8). Decreasing the IA concentration, increases MAA selectivity to 46 % with complete conversion of IA (Table 1 entry 9). In the gas phase analysis of the product mixture, carbon dioxide, carbon monoxide and propene were detected. Carbon dioxide and propene are the expected gaseous coproducts from decarboxylation of IA and MAA.

The absence of catalyst significantly decreases the yield of MAA. Notably, in a blank reaction only 28 % of MAA was formed with 87 % IA conversion (Table 1, entry 10).

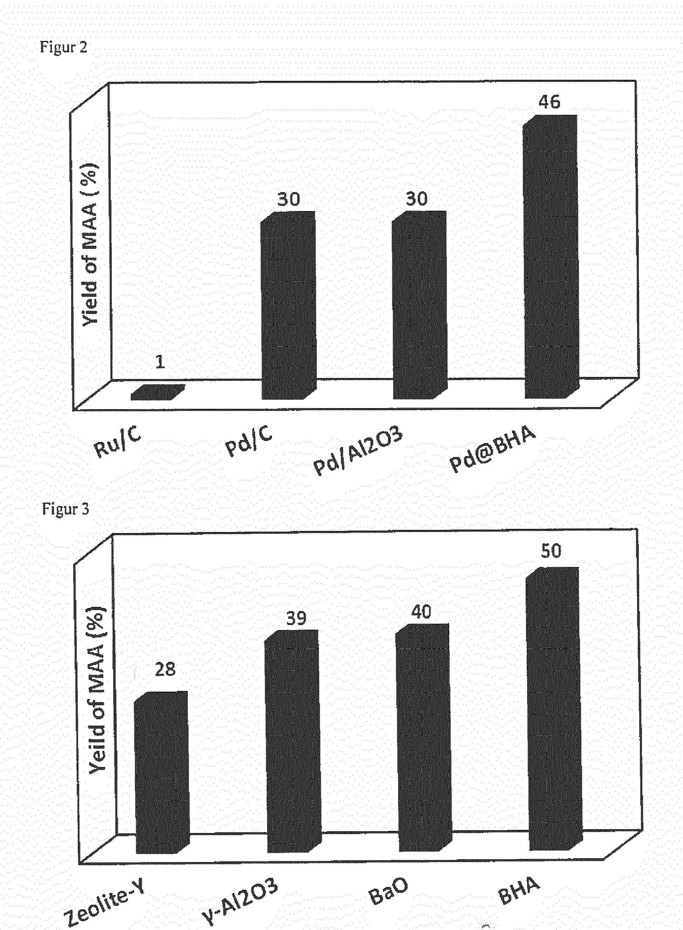

In order to prove the feasibility of the concept underlying the present invention, additional catalysts were compared with the activity of the Pd@BHA catalyst. Figure 2 shows that while the Ru/C catalyst showed a low activity it nevertheless enables the production of the target molecule while providing the possibility to separate the catalyst after the reaction from the reaction mixture without having to employ complicated methods. The selectivity for MAA was significantly increased (30%) employing a Pd/C catalyst and when using a Pd/Al₂O₃ catalyst.

**Table 2 summarizes the results obtained with another catalyst of the present invention.**

| **Table 2**. Screening of reaction conditions for decarboxylation of itaconic acid with BHA catalyst. | | | | | | | | | |
|---|---|---|---|---|---|---|---|---|---|
| Entry^{[a]} | IA[M] | P[bar] | Cat. [g] | t [h] | Conversion [%] | Selectivity [mol %] | | | |
| | | | | | | MAA | 2-HIBA | AA | Others^{[b]} |
| 1 | 0.10 | 20 | 1.0 | 3 | 100 | 50 | 18 | 3 | 29 |
| 2 | 0_15 | 20 | 1.0 | 3 | 100 | 45 | 18 | 1 | 36 |
| 3 | 0.10 | 30 | 1.0 | 3 | 100 | 45 | 19 | 2 | 33 |
| 4 | 0.10 | 20 | 3.0 | 3 | 100 | 42 | 23 | 1 | 34 |
| 5 | 0 10 | 20 | 10 | 4 | 100 | 49 | 23 | 4 | 24 |
| [a] Reaction conditions : Pressure reactors, IA, solvent (water), time (3 h), BHA catalyst, [b] Others = pyruvic acid, crotonic acid, acetone, propene, carbon dioxide and carbon monoxide. Values were determined by HPLC. IA is itaconic acid, MAA is methacrylic acid, AA is acetic acid and 2-HIBA is 2-hydroxyisobutyric. | | | | | | | | | |

First, decarboxylation of IA over a BHA catalyst was carried out under the optimized reaction conditions of the Pd@BHA catalyst (Table 1, entry 9). MAA was formed in very high yield (50 %) with complete conversion of IA (Table 2, entry 1). The BHA catalyst of the present invention was a highly efficient and selective for IA decarboxylation, and we thus commentaries 2 to 5 of table 2 show the influence of a number of reaction parameters on the MAA yield. The lower catalytic activity of the Pd@BHA catalyst compared to the BHA catalyst may be due to the low surface areas and high basicity of the Pd@BHA catalyst that promote fragmentation of IA and MAA. An increase of substrate concentration at constant temperature and pressure, decreases the selectivity of MAA to 45% and the yield of degradation products increased to 36 % (Table 2, entry 2). Similarly, no significant changes in MAA yield was observed at high pressure (Table 2, entry 3).

It was found that the MAA yield was decreased when the catalyst dosage was 3 g (Table 2, entry 4). Extending the reaction to 4 h shows only a marginal decrease in MAA yield (Table 2, entry 5). The screening experiments suggest that the reaction at 250 °C temperature and 20 bar pressure with 0.10 MIA and 1 g of BHA catalyst for 3 h offers the highest MAA yield (50 %) and low by-product formation.

Again, a comparison of the activity of the BHA catalyst with other catalysts was carried out. As shown in Figure 3, the framework-zeolite seems to be of lower activity for the decarboxylation reaction of IA, as same produced only 28 % of MAA. The moderate Lewis acidic γ-Al₂O₃ and highly Br⌀nsted basic barium oxide demonstrated higher activity than the zeolite (~40 %); however, this maximum yield is still significantly lower than that of BHA catalyst.

Additional proof for the superiority of the present invention was obtained when the catalytic effectiveness of the BHA catalyst was compared with the activity of the reported prior art catalysts. As depicted in Table 3, excellent yield of MAA (68 %) could be obtained when alkaline base and Pt/Al₂O₃ catalyst was utilized (entry 1). However, the alkaline base catalyst is corrosive and neutralization of the generated waste water stream is expensive. Additionally, platinum is an expensive noble metal that increase the total production cost. Another reaction using ruthenium carbonyl propionate as a homogenous catalyst with triphenylphosphine as an additive gave moderate yield of MAA (Table 3, entry 2). The results provided in table 3 (entry 3-5) demonstrate the superior catalytic activity of high surface area BHA catalysts and Si-Al catalyst compared to the other catalysts avoiding expensive transition metals and the use of liquid base.

| **Table 3.** A comparison of existing routes to bio-based MAA with the present work. | | | | | | |
|---|---|---|---|---|---|---|
| Entry^{[a]} | Catalysts | Temp. [°C] | Time | P [bar] | MAA Yield [%] | Ref. |
| 1 | Pt/Al₂O₃ and NaOH | 250 | 1 h | 38 | 68 | [ChemSusChem 2014, 7 (9), 2712-2720] |
| 2 | Ruthenium carbonyl propionate | 225 | 1.5 h | 28 | 34 | [ACS SUSTAIN CHEM ENG 2017, 5 (4), 3132-3140] |
| 3 | Pd@BHA | 250 | 3 h | 20 | 46 | invention |
| 4 | BHA | 250 | 3 h | 20 | 50 | invention |
| 5 | Si-Al-catalyst | 250 | 3 h | 20 | 46 | not according to the invention |
| [a] Reaction conditions : Reactor mode (batch), feedstock (IA), solvent (water). | | | | | | |

These results demonstrate that the preferred catalysts based on hexa-aluminate have a superior catalytic activity of (Pd@BHA, BHA) compared to the other catalysts. However, even the less active catalysts do show the possibility to produce MAA using heterogeneous catalysts, so that in particular the use of liquid bases as co-catalysts can be avoided. At the same time, compared with homogeneous catalysts employed in the prior art the present invention opens the way to re-use catalysts in an easy manner, as in particular the heterogeneous catalysts may be separated easily from the reaction mixture.

### Time Profile of decarboxylation with BHA and Pd@BHA catalysts

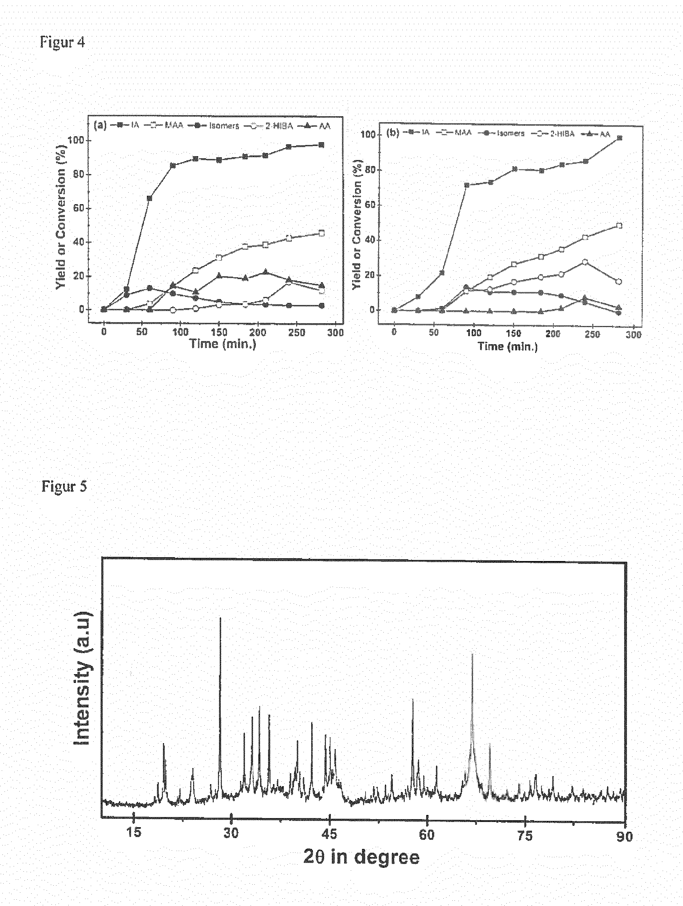

To understand what happens in the early stage of the reaction pathway toward MAA formation, especially isomerization and decarboxylation, investigations were made to determine the product distribution at various reaction times. The samples were collected at specific times and analyzed by HPLC. As shown in Figure 4a, the yield of MAA improved from 4% to 38% upon increasing the duration of the reaction from 60 minute to 184 minute with 91 % conversion of IA for Pd@BHA catalyzed decarboxylation of IA. Further increase in reaction time to 282 minute under similar conditions resulted in 46% MAA yield with 96 % IA conversion. Thus, the kinetics of MAA formation is rapid in first 184 minute followed by a slow reaction. A similar trend for the BHA catalyst was determined and is shown in Figure 4b.

It was observed that the rate of isomerization of IA was faster with the Pd@BHA catalyst compared with the BHA catalyst. 9 % of isomers were detected within 30 minute of reaction time at 139 °C for the Pd@BHA catalyst, whereas no isomers were detected for a BHA catalyzed reaction at same reaction time and temperature. As the reaction proceeds for 60 min, 13 % and 2 % isomers were formed at 236 °C with Pd@BHA and BHA catalysts, respectively. The high rate of isomerization of IA over Pd@BHA catalyst might be attributed to the high basicity of the catalyst which facilitates the dissociation of carboxylic acid to the corresponding carboxylate anions. The formation of isomers decreases gradually with time as the amount of MAA and side-products increases. The isomers mostly disappeared in 240 minutes for both the catalysts.

It was observed that the degradation of IA and MAA started after 90 minutes and 2-HIBA and AA were detected as the major by-products for both the catalysts. Notably, the total detected AA yield with Pd@BHA catalyst was much higher than that with BHA catalyst in all the samples. This may be due to the fact that the Pd species at the surface of the Pd@BHA catalyst might facilitate the radical fragmentation.

### EXAMPLE 1 Solid Base Catalyst

### Catalyst Synthesis

Barium hexa-aluminate (BHA) was prepared by the following procedure. 5.23 g of Ba(NO₃)₂ and 90.03 g of Al(NO₃)₃9H₂O were dissolved in 1000 mL deionized water at 60 °C, then 40 g CB (carbon black) was added and the mixture was vigorously stirred overnight to obtain a precursor slurry. Likewise, 96.09 g of (NH₄)₂CO₃ was dissolved in 200 mL deionized water and stirred for 1 h at 60°C. Subsequently, BHA the precursor slurry was poured into an (NH₄)₂CO₃ solution and the mixture was vigorously stirred for 4 h to obtain a gel. The obtained gel was filtrated, washed with deionized water and vacuum dried at 110°C overnight. The powders thus obtained were calcined in Ar at 1250 °C for 5 h followed by calcined at 900 °C in air for 12 h to entirely remove the carbon and to obtain barium hexa-aluminate.

Palladium supported barium hexa-aluminate (Pd@BHA) catalyst was prepared by a deposition-precipitation (DP) procedure. 1 g of BHA support was added to 20 mL of an aqueous solution of Pd(NO₃)₂. The pH of the solution was adjusted to 9 by adding 0.2 M NH₄OH under vigorous stirring. The mixture was then stirred for 1 h at 60 °C. After cooling to room temperature, the solid was recovered by filtration and washed with distilled water. The mixture was then placed in a vacuum oven and allowed to dry overnight at 40 °C. The dried material was then transferred to a Schwartz-type drying tube and reduced in H₂ at 350 °C for 3 h. The Pd@BHA catalyst was then cooled to RT under flowing N₂.

### Instrumentation

Powder X-ray diffraction (XRD) studies were conducted using the PANalytical X'Pert Pro instrument. Scanning from 10 to 90° was carried out using the CuKα radiation source with the wavelength of 0.15406 nm. Nitrogen physisorption analyses were carried out by degassing the catalysts under N2 flow for 4 h at 200 °C. The degassed samples were analyzed in the Micromeritics ASAP 2020 multi-point surface area and porosity analyzer.

Temperature programmed desorption (TPD) was performed using the Micromeritics 2920 Autochem II Chemisorption Analyzer. The catalysts were pre-treated at 350 °C under the stream of helium for 60 min. The temperature was consequently decreased to 80 °C. 9.8 mol. % CO₂ in He was passed over the catalysts at the flow rate of 30 mL min⁻¹ for 60 min. The excess gas was removed by purging with helium for 30 min. The temperature was after that gradually raised to 900 °C by ramping at 10 °C min⁻¹ under the flow of helium, wherein the desorption data of NH3, is recorded. The structure and morphology of the prepared catalysts was studied using field emission scanning electron microscope (SEM) (Carl Zeiss, Supra 35VP), equipped with energy-dispersive X-ray spectroscopy (EDXS) hardware (Oxford Instruments, INCA 400).

The amount of chemisorbed CO was measured with a pulse method using the Micromeritics 2920 Autochem II Chemisorption Analyser. The sample was first reduced with H2 at 400 °C for 1 h and then cooled to room temperature in flowing He. Several pulses of CO were introduced into the sample until no more adsorption was observed. The Pd dispersion (DPd) was calculated from the amount of CO chemisorption by assuming a stoichiometric ratio of CO/Pd = 1/1.

### Catalyst Characterization

### XRD Study:

The powder XRD pattern of the Pd@BHA catalyst is shown in Figure 5. In this pattern, an orthorhombic barium carbonate can be detected to be the major crystalline phase present with the peaks at a 2θ of 24.3°, 34.4° and 47.0° together with the boehmite (γ-AlOOH) phase at a 20 of peaks present at 14.7°, 28.8° and 38.5°. After loading with palladium, the BHA structure retains well and there are no specific peaks for metallic palladium, signifying that PdO phases are highly dispersed. The catalyst showed the characteristic lines of BaAl₂O₃ at a d-spacing of 3.2 and 4.5 Å, having relative intensities of 55 and 18 % respectively. Besides barium mono aluminate, the other major phases present are γ-Al₂O₃ (20 = 66.3°, 45.8° and 36.8°). In addition to this, a β-phase of BaAl₂O₃ is also observed with the 20 at 19.8°, 31.5°, 34.2° and 57.8° having relative intensities of 12 %. There is crystalline phase of Al is observed in the catalyst, suggesting that aluminum oxo-hydroxide might be present in an amorphous or non-crystalline form.

### Surface properties and basicity measurement:

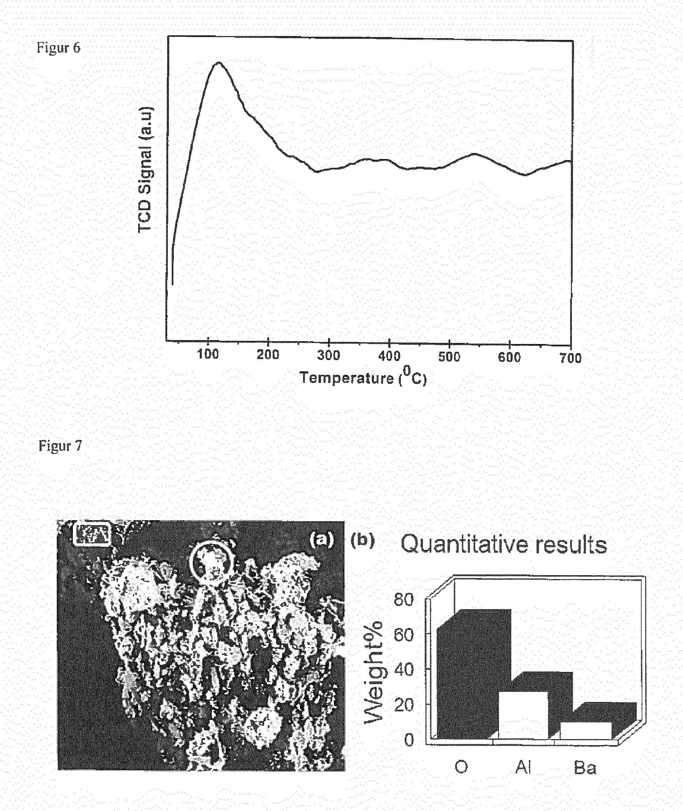

The Pd@BHA catalyst is characterized by the highest specific surface area (52.4 m²/g) and pore volume (0.40 cm³/g). The basicity was determined by CO₂-TPD and the profiles of the Pd@BHA catalyst exhibited only a large peak of CO₂ desorption. CO₂ is a weak acidic molecule and can be adsorbed on weak and strong basic sites. Figure 6 shows the desorption profile of CO₂ of the Pd@BHA catalyst. The weak, strong and some moderate basic sites were ascribed to the three peaks, namely the weak basic sites at 150-300 °C, the moderate basic sites at 300-600 °C, and the strong basic sites above 600 °C, resepctively. In general, weak basic sites are attributed to alumina and barium, and are modified by the presence of Pd on the surface of the prepared catalysts. In multicomponent mixed oxides, a simpler situation is represented by the case, in which only two types of cations are present. When the two cations have similar oxidation states and electronegativity, acidic-alkaline characteristics are often dominated by one of the cations. Thus, in the present case, the basic character of the Pd@BHA catalyst is strongly pronounced by the presence of Pd when compared to Ba and Al. The amount of CO₂ desorbed was taken as measures for weak, medium and strong basic sites with corresponding base density values of 0.00464 mmol g⁻¹.

### Nanostructural Analysis:

Figure 7a shows the fine intermixing of two main phases of BaO, having irregular block shaped morphology (marked red) and boehmite (having fabric morphology, marked green) which are visible in the micrograph. It should be noted that the catalyst is calcined at a high temperature and still attained the boehmite morphology. It is evident that Pd is well dispersed on the surface of the support which showed an amorphous morphology. To identify the dispersion of Pd on the support an EDS (pattern shown in Figure 7b) on this catalyst is carried out and showed the qualitative presence of barium, aluminums and palladium.

### EXAMPLE 2 Catalytic Decarboxylation Reactions and analysis

### Conversion of itaconic acid to methacrylic acid:

Decarboxylation reactions were performed in a high pressure batch reactor (250 mL, Amar Equipment Pvt. Ltd., India) equipped with a thermocouple, pressure gauge, rupture disk, and gas release valve. In a typical experiment, desired amount of biomass derived feedstock was dissolved in deionized water. The solution was loaded into the autoclave reactor with catalyst and sealed. The reactor was pressurized with N₂ to 10 bar and vented three times to remove any residual oxygen atmosphere. Finally the reactor was pressurized to desired pressure, stirred (600 rpm) and continued heating at desired temperature for desired time. Once the reaction was completed the reactor was cooled to room temperature. After collecting gas and liquid samples, headspace pressure was released. The crude reaction mixture was analyzed by HPLC.

### Recyclability Study of the catalysts:

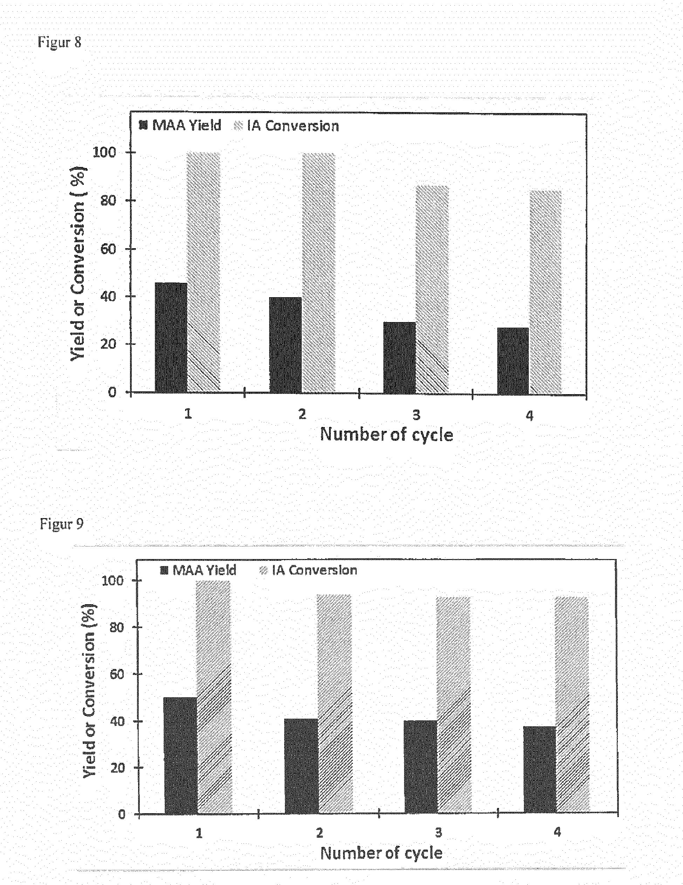

The recycling efficiency of the catalyst was determined for the decarboxylation of IA as a representative reaction. In this study, an aqueous solution of itaconic acid and the catalyst were placed in a high pressure reactor for the desired time, and at desired temperatures and pressures. After each cycle, the catalyst was recovered, washed with distilled water, dried and reused for the next cycle by adding fresh IA and water. Fresh catalyst was not added to replenish any loss of the catalyst mass during recovery. The yield of MAA was determined from each run and Figures 8 and 9 display the relevant results

### Determination of MAA Yield:

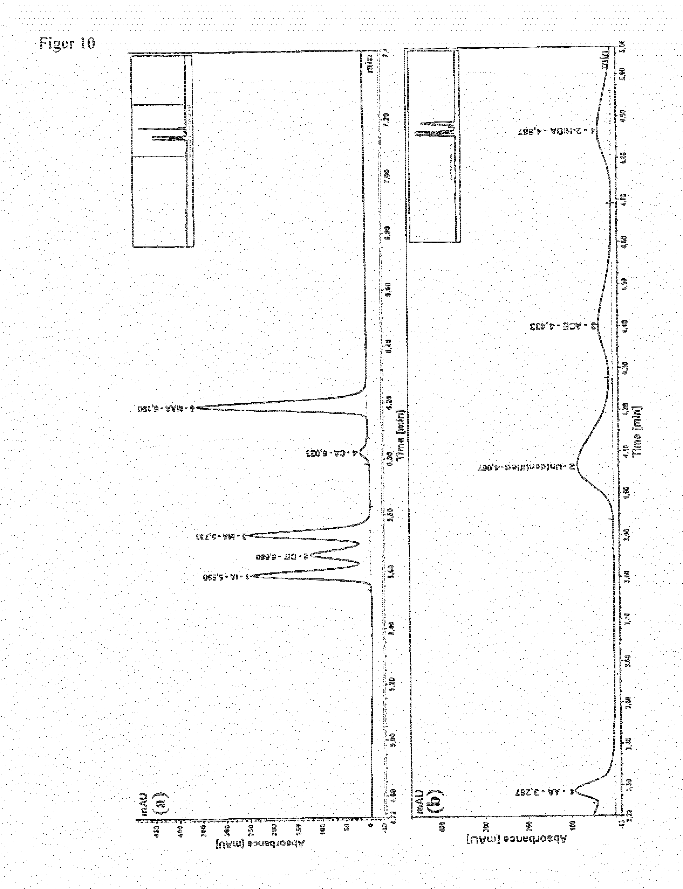

HPLC analysis was performed by using Ultra-high-pressure liquid chromatography - UHPLC (Thermo-Fisher Scientific UltiMate^{™} 3000) equipped with an Acclaim^{™} Organic Acid LC Column (5.0 µm; 4× 25 mm) heated to 45°C and a DAD detector. Compounds listed in Table (S1) were identified by retention time and UV-Vis spectra comparison to reference standards using both ultraviolet detections at a wavelength of 210 nm and the UV-VIS specter from 205 nm - 400 nm. Individual compounds were quantified by prepared calibration standards. The mobile phase with a flow rate 1 ml/min consisted of an aqueous phase (2.5 mM H₂SO₄ solution with pH 4) and organic phase (acetonitrile) with a flow rate of 1.0 mL/min. A1 or 50 µL of the sample was injected and a gradient method, starting with 100% of aqueous phase (2.5 mM H₂SO₄ at pH 2.4) with addition of organic phase (acetonitrile) for 5 minutes to reach 90% of acetonitrile and kept it for 3 min, the gradient was reversed to reach again 100% of aqueous phase at 9 min, which was kept until 10 min. Figure 10 shows the HPLC chromatogram of the reaction mixture. All the peaks shown in the chromatogram were identified and quantified. The following retention times were confirmed: for acetic acid (3.26 min), acetone (4.5 min), 2-Hydroxyisobutaric acid (4.84 min), itaconic acid (5.62 min), citraconic acid (5.70 min), mesaconic acid (5.76 min), crotonic acid (6.05 min) and methacrylic acid (6.22 min).

## Claims

1. Process for the production of methacrylic acid (MAA) comprising the steps:
**(a)** feeding a mixture comprising a starting material and a heterogeneous catalyst into a reactor,
**(b)** heating said mixture at a temperature ranging from 150°C to 300 °C and at a pressure ranging from 5 to 100 bar for a time period ranging (reaction time / residence time) from 30 to 1000 minutes to obtain crude methacrylic acid,
**(c)** and separating the crude methacrylic acid from the catalyst,
wherein the starting material is selected among carboxylic acids from at least one of citric acid, aconitic acid, itaconic acid, mesaconic acid, and citraconic acid, preferably obtained from renewable resources,
**(d)** and wherein the catalyst is a heterogeneous catalyst comprising a hexa-aluminate, wherein no liquid bases as co-catalyst are employed.

2. The process as claimed in claim 1, wherein the mixture further comprises a polar solvent, preferably water.

3. The process as claimed in at least one of the preceding claims, wherein the amount of catalysts ranges from 10 to 150 mass percentages with respect to substrate.

4. The process as claimed in any of the preceding claims, wherein the concentration of bio-based carboxylic acid is in the range of from 0.10M to 0.5M.

5. The process according to any one of the preceding claims, wherein the temperature is from 200°C to 250°C, the pressure is from 10 to 30 bar and the time period is from 80 to 200 min.

6. The process according to any one of the preceding claims, wherein the catalyst is barium hexa-aluminate (BHA) or Pd supported on BHA.

7. Use of a catalyst in the process of claim 1 for the production of MAA from carboxylic acids, selected from at least one of citric acid, aconitic acid, itaconic acid, mesaconic acid, and citraconic acid, preferably obtained from renewable resources,wherein the catalyst is a heterogeneous catalyst based on hexa-aluminate.

8. The use according to claim 7, wherein the catalyst has a BET surface area of from 1 to 100 m² and/or a pore volume of from 0.1 to 0. 50 cm³.

9. The use according to at least one of claims 7 to 8, wherein the catalyst is selected among BHA and Pd supported on BHA.

## Patentansprüche

1. Verfahren zur Herstellung von Methacrylsäure (MAA), umfassend die Schritte:
**(a)** Einspeisen einer Mischung, die ein Ausgangsmaterial und einen heterogenen Katalysator enthält, in einen Reaktor,
**(b)** Erhitzen des Gemisches bei einer Temperatur von 150°C bis 300°C und einem Druck von 5 bis 100 bar über einen Zeitraum (Reaktionszeit / Verweilzeit) von 30 bis 1000 Minuten, um rohe Methacrylsäure zu erhalten,
**(c)** und Abtrennen der rohen Methacrylsäure von dem Katalysator,
wobei das Ausgangsmaterial unter Carbonsäuren aus mindestens einer der folgenden Substanzen ausgewählt wird: Zitronensäure, Aconitsäure, Itaconsäure, Mesaconsäure und Citraconsäure, die vorzugsweise aus erneuerbaren Ressourcen gewonnen werden,
**(d)** und wobei der Katalysator ein heterogener Katalysator ist, der ein Hexa-Aluminat umfasst, wobei keine flüssigen Basen als Co-Katalysator verwendet werden.

2. Verfahren nach Anspruch 1, wobei das Gemisch ferner ein polares Lösungsmittel, vorzugsweise Wasser, enthält.

3. Verfahren nach mindestens einem der vorhergehenden Ansprüche, wobei die Menge an Katalysatoren im Bereich von 10 bis 150 Massenprozent, bezogen auf das Substrat, liegt.

4. Verfahren nach einem der vorangehenden Ansprüche, wobei die Konzentration der biobasierten Carbonsäure im Bereich von 0,10 M bis 0,5 M liegt.

5. Verfahren nach einem der vorangehenden Ansprüche, wobei die Temperatur 200°C bis 250°C, der Druck 10 bis 30 bar und die Zeitdauer 80 bis 200 min beträgt.

6. Verfahren nach einem der vorhergehenden Ansprüche, wobei der Katalysator Bariumhexaaluminat (BHA) oder Pd unterstützt auf BHA ist.

7. Verwendung eines Katalysators in dem Verfahren nach Anspruch 1 zur Herstellung von MAA aus Carbonsäuren, ausgewählt aus mindestens einer der Säuren Zitronensäure, Aconitsäure, Itaconsäure, Mesaconsäure und Citraconsäure, die vorzugsweise aus nachwachsenden Rohstoffen gewonnen werden, wobei der Katalysator ein heterogener Katalysator auf Basis von Hexa-Aluminat ist.

8. Verwendung nach Anspruch 7, wobei der Katalysator eine BET-Oberfläche von 1 bis 100m² und/oder ein Porenvolumen von 0,1 bis 0,50 cm³ aufweist.

9. Verwendung nach mindestens einem der Ansprüche 7 bis 8, wobei der Katalysator ausgewählt ist aus BHA und Pd unterstützt auf BHA.

## Revendications

1. Procédé de production d'acide méthacrylique (MAA) comprenant les étapes suivantes :
(a) introduire un mélange comprenant un matériau de départ et un catalyseur hétérogène dans un réacteur,
(b) chauffer ledit mélange à une température allant de 150 °C à 300 °C et à une pression allant de 5 à 100 bars pendant un laps de temps (temps de réaction/temps de séjour) allant de 30 à 1 000 minutes afin d'obtenir de l'acide méthacrylique brut,
(c) et séparer l'acide méthacrylique brut du catalyseur,
dans lequel le matériau de départ est choisi parmi des acides carboxyliques parmi au moins l'un de l'acide citrique, l'acide aconitique, l'acide itaconique, l'acide mésaconique et l'acide citraconique, obtenus de préférence à partir de ressources renouvelables,
(d) et dans lequel le catalyseur est un catalyseur hétérogène comprenant un hexa-aluminate,
dans lequel aucune base liquide n'est employée en tant que co-catalyseur.

2. Procédé tel que revendiqué dans la revendication 1, dans lequel le mélange comprend en outre un solvant polaire, de préférence l'eau.

3. Procédé tel que revendiqué dans l'une au moins des revendications précédentes, dans lequel la quantité des catalyseurs varie de 10 à 150 pour cent en masse par rapport au substrat.

4. Procédé tel que revendiqué dans l'une quelconque des revendications précédentes, dans lequel la concentration d'acide carboxylique d'origine biologique est dans la plage allant de 0,10M à 0,5M.

5. Procédé selon l'une quelconque des revendications précédentes, dans lequel la température va de 200 °C à 250 °C, la pression va de 10 à 30 bars et le laps de temps va de 80 à 200 min.

6. Procédé selon l'une quelconque des revendications précédentes, dans lequel le catalyseur est l'hexa-aluminate de baryum (BHA) ou du palladium supporté sur BHA.

7. Utilisation d'un catalyseur dans le procédé selon la revendication 1 pour la production de MAA à partir d'acides carboxyliques, choisis parmi l'un au moins de l'acide citrique, l'acide aconitique, l'acide aconitique, l'acide itaconique, l'acide mésaconique et l'acide citraconique, de préférence obtenus à partir de ressources renouvelables, dans laquelle le catalyseur est un catalyseur hétérogène basé sur l'hexa-aluminate.

8. Utilisation selon la revendication 7, dans laquelle le catalyseur a une surface active BET allant de 1 à 100 m² et/ou un volume de pores allant de 0,1 à 0,50 cm³.

9. Utilisation selon l'une au moins des revendications 7 à 8, dans laquelle le catalyseur est choisi parmi le BHA et le palladium supporté sur BHA.
